# EUROPÄISCHE PATENTSCHRIFT

(11) **EP 1 935 362 B1**
(45) Veröffentlichungstag und Bekanntmachung des Hinweises auf die Patenterteilung: **06.04.2011**
(21) Anmeldenummer: 06026421.5
(22) Anmeldetag: 20.12.2006
(51) Int. Cl.: A61B 19/00

(54) **Markersystem zum Bestimmen von Durchmesser und Achslage eines Lochs in einem Instrument**
Marker system to determine the diameter and alignment of a hole within an instrument
Système de marquage pour déterminer le diamètre et l' orientation d'un trou dans un instrument

(43) Veröffentlichungstag der Anmeldung: 25.06.2008
(73) Patentinhaber: BrainLAB AG, 85622 Feldkirchen (DE)
(72) Erfinder: Bogojevic, Aleksander, 81677 München (DE); Maier, Christian, 80802 München (DE); Maier, Georg, 81541 München (DE); Uhde, Jörg, 80538 München (DE)
(74) Vertreter: Schwabe - Sandmair - Marx

(56) Entgegenhaltungen:
- WO-A-02/061371
- WO-A-2006/029541
- US-A1- 2004 054 489

## Beschreibung

Die vorliegende Erfindung betrifft ein Markersystem, das die Bestimmung von Durchmesser und Achslage von Löchern in Gegenständen, insbesondere Instrumenten oder Körperstrukturen (z.B. Knochen) ermöglicht. Insbesondere erlaubt es die Bestimmung der Lage der Achse eines rotationssymmetrischen Loches bzw. einer Bohrung in einem Instrument, insbesondere medizinischen Instrument. Rein beispielhaft wird im Folgenden von einem Instrument anstelle von einem Gegenstand gesprochen.

Gegenwärtig werden mittels einer Detektionseinrichtung (z. B. Kamera oder Ultraschalldetektor) Markereinrichtungen detektiert. Bei den Markereinrichtungen handelt es sich typischerweise um drei Marker, die in fester und vorbestimmter relativer Lage zueinander angeordnet sind und insbesondere mechanisch verbunden sind. Die Marker können passive oder aktive Marker sein, wobei die passiven Marker Signale (z.B. Wellen und/oder Strahlung) reflektieren, die in ihre Richtung ausgesendet werden und die aktiven Marker selbst Ursprung der Signale (z.B. Strahlung und/oder Wellen) sind. Die von den (aktiven oder passiven) Markern ausgehenden Signale, bei denen es sich um Wellensignale oder Strahlungssignale handeln kann, werden von einer Detektionseinrichtung (z. B. Kamera) detektiert. Um eine Position der Markereinrichtung relativ zu der Detektionseinrichtung festzulegen, wird dabei vorzugsweise die Markereinrichtung bewegt, um der Detektionseinrichtung verschiedene Ansichten der Markereinrichtung zu bieten. Auf dieser Grundlage kann dann in bekannter Weise die relative Lage der Markereinrichtung relativ zu der Detektionseinrichtung, insbesondere in einem im Raum ruhenden Bezugssystem bestimmt werden. In diesem Zusammenhang wird auf die DE 196 39 615 A1 und die entsprechende US-Veröffentlichung 6,351,659 hingewiesen. Dokument WO 2006/029541 offenbart eine Vorrichtung zum kalibrieren von geometrischen Abmessungen, insbesondere des Durchmessers, sowie Lage und/oder Orientierung von Instrumenten im Raum.

Aufgabe der Erfindung ist es, die vorgenannten Markereinrichtungen zu nutzen, um möglichst effizient, insbesondere in einem Arbeitsschritt bzw. Kalibrationsschritt, sowohl die Lage der Achse eines Lochs in einem Instrument als auch ein für das Loch typisches Kenndatum bzw. typische Kenndaten, die die Ausformung des Lochs charakterisieren, zu bestimmen.

Vorstehende Aufgabe ist durch die Gegenstände der unabhängigen Ansprüche gelöst. Vorteilhafte Ausführungsformen gehen aus den Unteransprüchen hervor.

Erfindungsgemäß ist ein Markersystem vorgesehen, das die Bestimmung des Durchmessers und der Achslage eines rotationssymmetrischen Loches in einem Instrument erlaubt. Der Begriff "rotationssymmetrisch" bezieht sich dabei auf die Achse des Loches, d.h. die Mantelfläche, die das Loch umgibt und begrenzt, hat eine Form, die bezüglich der Achse in Längsrichtung (Eindringrichtung in das Loch) rotationssymmetrisch ist. Beispiele für rotationssymmetrische Löcher sind zylindrische Löcher (Bohrungen oder Durchgangsbohrungen) aber beispielsweise auch kegelförmige oder konusförmige oder teil-sphärisch gestaltete Löcher (z.B. Halbkugeln). Der Begriff "Loch" hierin umfasst sowohl Durchgangslöcher, die das Instrument durchdringen, als auch eine Vertiefung in dem Instrument. Das Markersystem umfasst einen Körper, an dem Marker angebracht sind, die im Folgenden "Körper-Marker" genannt werden. Die Körper-Marker befinden sich in einer vorbestimmten Anordnung relativ zueinander und weisen insbesondere eine vorbekannte Größe und insbesondere Form (z.B. Kugelform) auf. Der Körper weist somit eine Markereinrichtung auf. Vorzugsweise weist auch das Instrument eine Markereinrichtung auf, wie weiter unten noch näher ausgeführt wird. Für Markereinrichtungen gilt allgemein Folgendes:

Die Lage der Markereinrichtung wird bevorzugt durch die Position der Markereinrichtung in einem vorbestimmen Bezugssystem bestimmt. Vorzugsweise wird als Bezugssystem ein Bezugssystem verwendet, in dem die Detektionseinrichtung ruht. Insbesondere wird die Lage der Markereinrichtung durch die Positionen der Marker, insbesondere der Mittelpunkte der Marker in einem Bezugssystem bestimmt. Die Positionen können zum Beispiel mit kartesischem Koordinaten oder Kugelkoordinaten beschrieben werden. Die relative Lage von einem Teil (z. B. Detektionseinrichtung oder Markereinrichtung) zu einem anderen Teil (z. B. Markereinrichtung) kann insbesondere durch Raumwinkel und/oder Abstände und/oder Koordinaten (in einem Bezugssystem) und/oder Vektoren beschrieben werden und wird vorzugsweise aus dem die Lage beschreibenden Positionen z. B. mittels eines Programms errechnet, das auf einem Computer läuft.

Der hierin verwendete Begriff "relative Lage" oder der Ausdruck "Lage eines Teils A relativ zu einem Teil B" umfasst also den Begriff der relativen Positionen zwischen den zwei Teilen, insbesondere zwischen den Markereinrichtungen und/oder deren Markern oder zwischen einer Markereinrichtung (oder deren Markern) und der Detektionseinrichtung. Insbesondere werden Schwerpunkte oder Mittelpunkte der Teile als punktförmiger Bezugspunkt zum Festlegen einer Position gewählt. Ist die Position eines Teils in einem Bezugssystem bekannt, so kann basierend auf der relativen Lage zweier Teile aus der Position eines der zwei Teile die Position des anderen der zwei Teile berechnet werden.

Insbesondere falls die Markereinrichtung nur zwei Marker umfasst, ist bevorzugt eine Startposition bekannt und das Markersystem erlaubt dann die Verfolgung der- Lage der Markereinrichtung bei Bewegung der Markereinrichtung im Raum.

Die Markereinrichtung gemäß der vorliegenden Erfindung umfasst also vorzugsweise mindestens zwei Marker, insbesondere und bevorzugt drei Marker. Die Abmessungen der Marker und die relative Lage der Marker zueinander ist bekannt und liegt insbesondere als vorbekannte Daten einer Datenverarbeitungseinrichtung vor. Vorzugsweise ist auch die Form der Marker bekannt.

Weiter umfasst das erfindungsgemäße Markersystem vorzugsweise eine Detektionseinrichtung, die Signale von den mindestens zwei Markern detektiert. Wie zuvor ausgeführt handelt es sich hierbei um von den Markern ausgehende Signale, die entweder aktiv von den Markern ausgesendet werden oder von den Markern reflektiert werden. Im letzteren Fall ist vorzugsweise weiter eine Signalsendequelle beispielsweise eine Infrarotlichtquelle vorgesehen, die Signale (z. B. Ultraschallpulse oder Infrarotlicht) in Richtung auf die passiven Marker aussendet, wobei die passiven Marker die Signale reflektieren. Eine Datenverarbeitungseinrichtung, insbesondere ein Computer erlaubt die Berechnung der relativen Lage der Markereinrichtung relativ zur Detektionseinrichtung, insbesondere die Berechnung der Lage der Markereinrichtung in einem Bezugssystem, in dem die Detektionseinrichtung ruht, also beispielsweise in einem Bezugssystem, das in einem Operationssaal ruht.

Ist nun der Körper mit einer Markereinrichtung versehen und auch das zu untersuchende Instrument mit einer Markereinrichtung versehen, so lässt sich somit die relative Lage des Körpers relativ zu dem Instrument bestimmen. Unbekannt ist jedoch noch die Lage der Achse des Loches relativ zu der am Instrument angebrachten Markereinrichtung sowie die Ausformung des Loches.

Um dies zu bestimmen sind nicht nur Körper-Marker an dem Körper vorgesehen sondern auch rotationssymmetrische Fortsätze. Diese können also beispielsweise eine zylindrische Gestalt haben oder eine kegelförmige Gestalt (einschließlich eines Kegelstumpfes).

Ein Bediener, der das Loch in einem (beliebigen) Instrument vermessen möchte, nimmt sich ein Instrument, das mit einer Markereinrichtung versehen ist. Die Marker der Markereinrichtung werden hierin als "Instrumenten-Marker" bezeichnet, um sie von den Körper-Markern zu unterscheiden. Der Bediener sucht sich denjenigen Fortsatz, der in das Loch des Instruments möglichst passgenau passt und dreht das Instrument um dieses Loch, während die Signale, die von den Instrumentenmarkern ausgehen, von der Detektionseinrichtung erfasst werden. Aufgrund der Ausgestaltung des erfindungsgemäßen Markersystems erlaubt diese Vorgehensweise die Bestimmung sowohl der Ausformung des Lochs (also beispielsweise des Durchmessers eines zylindrischen Loches) als auch der Lage der Achse des Loches.

Die erfindungsgemäße Detektionseinrichtung ist zur Detektion von Signalen ausgestaltet, die sowohl von den Körper-Markern als auch von den Instrumenten-Markern ausgehen. Dies erlaubt der Datenverarbeitungseinrichtung, die relative Lage der Körper-Marker relativ zu den Instrumenten-Markern zu bestimmen. Um diese Bestimmung und/oder andere Bestimmungen durchzuführen, wandelt vorzugsweise die Detektionseinrichtung die detektierten Signale in Datensignale um und gibt sie an die Datenverarbeitungseinrichtung aus. Die erfindungsgemäße Datenverarbeitungseinrichtung ist vorzugsweise ausgebildet, die im Folgenden näher beschriebenen Funktionen des Empfangens, Speichems, Berechnens und Bestimmens auszuführen. Die Datenverarbeitungseinrichtung empfängt die Datensignale, die von der Detektionseinrichtung ausgegeben werden. Dies bedeutet, sie empfängt Datensignale, die die relative Lage der Körper-Marker relativ zur Detektionseinrichtung darstellen und Datensignale, die die relative Lage der Instrumentenmarker relativ zur Detektionseinrichtung darstellen. Wie bereits zuvor ausgeführt, wird von einem Bediener vorzugsweise das Instrument um einen Fortsatz gedreht, der in das Loch des Instruments eingepasst wurde. Somit ergeben sich mehrere relative Lagen der Instrumenten-Marker relativ zu der Detektionseinrichtung während dieser Drehbewegung. Erfindungsgemäß sind vorzugsweise wenigstens drei relative Lagen der Instrumenten-Marker relativ zur Detektionseinrichtung vorgesehen. Die Datenverarbeitungseinrichtung ist deshalb ausgebildet, mindestens drei verschiedene relative Lagen der Instrumenten-Marker relativ zur Detektionseinrichtung zu empfangen und weiterzuverarbeiten. Bei der weiteren Verarbeitung der verschiedenen relativen Lagen der Instrumenten-Marker (relativ zur Detektionseinrichtung) wird dann bevorzugt angenommen, dass diese relativen Lagen auf einer Kreisbahn liegen. Vorzugsweise wird ein Annährungsverfahren (Fittingverfahren wie beispielsweise Least-Square-Fit) durchgeführt, um aus den drei relativen Lagen eine Kreisbahn zu berechnen, auf der sie zumindest in etwa liegen. Die Datenverarbeitungseinrichtung hat somit die Funktion der Berechnung einer Kreisbahn und der Annahme, dass die mindestens drei, verschiedenen relativen Lagen auf einer Kreisbahn liegen. Zur mathematischen Beschreibung einer Kreisbahn wird vorzugsweise die Lage des Mittelpunktes und der Radius der Kreisbahn verwendet sowie insbesondere trigonometrische Funktionen (z.B. Sinus, Kosinus). Somit ist mit der Berechnung der Kreisbahn auch die Lage des Mittelpunkts der Kreisbahn berechnet und relativ zur Detektionseinrichtung bestimmt. Da die Lage des Mittelpunkts der Kreisbahn mit der Lage der Achse des Loches im Instrument übereinstimmt, ist somit auch die relative Lage der Achse des Loches relativ zur Detektionseinrichtung bestimmt.

Der zuvor erwähnte Arbeitsschritt des Einpassens eines Fortsatzes in ein Instrumentenloch mit anschließendem Drehen des Instruments um diesen Fortsatz erlaubt aber nicht nur die Bestimmung der relativen Lage der Achse des Loches sondern auch die Bestimmung der Ausformung des Loches. Die Ausformung des Loches wird bevorzugt durch ein Kenndatum oder mehrere Kenndaten beschrieben. Im Falle eines zylindrischen Loches und somit eines zylindrischen Fortsatzes ist dieses mindestens eine Kenndatum z.B. der Durchmesser oder Radius des Loches. Beispielsweise zusätzlich auch noch die Höhe des zylindrischen Fortsatzes. Es können auf dem Körper aber auch rotationssymmetrische Fortsätze unterschiedlicher Ausformung vorgesehen sein, also beispielsweise zylindrisch ausgeformte, kegelförmig ausgeformte oder sphärisch ausgeformte Fortsätze. In diesem Fall umfasst das mindestens eine Kenndatum vorzugsweise auch noch ein Kenndatum, das auf die Gestalt hinweist, also beispielsweise zylindrisch, kegelförmig oder sphärisch. Handelt es sich beispielsweise nur um kegelförmig gestaltete Fortsätze mit einer Kegelspitze (also keinen Kegelstumpf), so umfasst das mindestens eine Kenndatum vorzugsweise den Öffnungswinkel des Kegels. Bei einem Kegelstumpf wäre z.B. die Höhe des Kegelstumpfes und/oder die Radien bzw. Durchmesser der den Kegelstumpf abschließenden Flächen weitere Kenndaten.

Erfindungsgemäß ist die Datenverarbeitungseinrichtung ausgebildet, um das mindestens eine Kenndatum zu bestimmen. Hierzu sind vorzugsweise die relativen Lagen zwischen den Körper-Markern und den Achsen der rotationssymmetrischen Fortsätze bekannt und in der Datenverarbeitungseinrichtung gespeichert. Weiter sind zugeordnet zu den vorgenannten relativen Lagen der Achsen relativ zu den Körper-Markern die vorgenannten Kenndaten gespeichert. Insbesondere ist zugeordnet zu jeder einzelnen Achse mindestens ein Kenndatum gespeichert.

Somit lässt sich aus den Datensignalen berechnen, mit welcher der Achsen der berechnete Mittelpunkt übereinstimmt. Insbesondere lässt sich die relative Lage des Mittelpunkts und damit der Achse relativ zu der Detektionseinrichtung bestimmen. Weiter lässt sich aus der detektierten Lage der Körper-Marker und aus den gespeicherten Daten über die relative Lage der Körper-Marker relativ zu den Achsen bestimmen, mit welcher der Achsen der Mittelpunkt übereinstimmt. Somit erlaubt die Detektion der Instrumenten-Marker die Bestimmung der relativen Lage des Mittelpunktes der Kreisbahn relativ zur Detektionseinrichtung. Weiter erlaubt die Detektion der Körper-Marker die Bestimmung der relativen Lage der Achsen relativ zur Detektionseinrichtung. Man kann also bestimmen, welche der jeweiligen, bestimmten relativen Lagen der Achsen relativ zur Detektionseinrichtung mit der bestimmten relativen Lage des Mittelpunkts relativ zur Detektionseinrichtung übereinstimmt. Somit kann man bestimmen, mit welcher Achse der Mittelpunkt übereinstimmt, um so die Achse zu identifizieren. Ist nun die Achse identifiziert, so lässt sich aus der gespeicherten Zuordnung zwischen Achse und Kenndatum das der identifizierten Achse zugeordnete Kenndatum bestimmen. Somit kann durch den vorgenannten Arbeitsschritt sowohl die relative Lage der Achse zu der Detektionseinrichtung und damit beispielsweise auch relativ zu den Instrumenten-Markern bestimmt werden. Außerdem kann gleichzeitig das Kenndatum des Loches im Instrument bestimmt werden. Das Instrument ist also registriert und kann beliebig eingesetzt werden. Insbesondere kann für eine spätere Verwendung des Instruments die bestimmte relative Lage der Achse des Loches relativ zu den Instrumenten-Markern und das mindestens eine Kenndatum, das die Ausformung des Loches charakterisiert, abgespeichert werden.

Wie bereits zuvor ausgeführt, ist die Anordnung der Körper-Marker relativ zueinander bekannt und vorzugsweise abgespeichert und unterscheidet sich insbesondere von der vorzugsweise ebenfalls abgespeicherten Anordnung und gegebenenfalls auch Ausformung der Instrumenten-Marker. Vorzugsweise sind alle Marker kugelförmig. Aufgrund der jeweils charakteristischen Anordnung der Körper-Marker und charakteristischen Anordnung der Instrumenten-Marker ist es möglich, die Körper-Marker von den Instrumenten-Markern zu unterscheiden. Somit kann die Datenverarbeitungseinrichtung bei der Verarbeitung der Datensignale erkennen, welche Datensignale den Körper-Markern zuzuordnen sind und welche den Instrumenten-Markern.

Vorzugsweise ist der Abstand zwischen zwei Fortsätzen größer als der Durchmesser eines der Fortsätze, insbesondere größer als der maximale Durchmesser der Fortsätze. Ein bevorzugter Bereich liegt bei 100 bis 300 % des Durchmessers.

Die Oberfläche, aus der die Fortsätze hervorstehen, ist vorzugsweise konvex gekrümmt oder plan, um ein Anlegen des Instruments direkt an die Oberfläche zu ermöglichen und gleichzeitig ein Drehen des Instruments um den Fortsatz, wenn der Fortsatz in das Loch des Instruments eingeführt wurde, zu ermöglichen und nicht zu behindern. Eine plane Ausführung der Oberfläche wird bevorzugt, da somit auch eine seitliche Führung des Instruments während der Drehbewegung um die Achse des Fortsatzes erzielt werden kann. Die Schnittpunkte der Achse der Fortsätze mit der vorgenannten Oberfläche stellen vorzugsweise Punkte dar, die auf einer Trajektorie liegen. Die Trajektorie ist beispielsweise eine Gerade oder eine gekrümmte Linie, insbesondere eine geschlossene Linie (beispielsweise Kreis oder Ellipse). Durch diese Anordnung wird erreicht, dass ein Drehen des Instruments um eine der Achsen der Fortsätze nicht durch andere Fortsätze behindert wird.

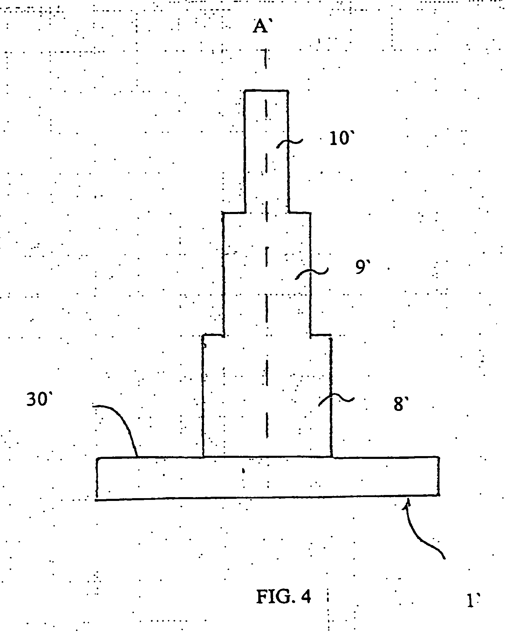

Alternativ oder zusätzlich können die Fortsätze auch übereinander angeordnet sein, so dass die Achsen der Fortsätze ineinander übergehen, wobei sich der Durchmesser der Fortsätze mit zunehmendem Abstand von der vorgenannten Oberfläche verringert. Die Verringerung des Durchmessers erfolgt vorzugsweise stufenweise (siehe Figur 4).

Der Körper, an dem die Fortsätze vorgesehen sind, kann auch mehrteilig ausgebildet werden. Insbesondere kann ein Teil des Körpers, der hierin als "Fortsatz-Körper" bezeichnet wird, und an dem die Fortsätze vorgesehen sind, von einem anderen Teil des Körpers, der hierin als ,,Markereinheit" bezeichnet wird, getrennt sein. Eine Markereinrichtung ist bei der Markereinheit vorgesehen. Auf diese Art und Weise lassen sich verschieden gestaltete Markereinrichtungen mit dem Fortsatz-Körper verbinden, um so eine für den jeweiligen Anwendungszweck geeignete Markereinrichtung als Körper-Marker einsetzen zu können. Andererseits lässt sich natürlich mit einer bestimmten Markereinrichtung mehrere verschiedene Fortsatz-Körper verbinden. So kann man beispielsweise je nach Ausformung des Loches einen Fortsatz-Körper mit zylindrischen Fortsätzen oder einen mit konischen Fortsätzen auswählen oder man kann Fortsatzkörper mit unterschiedlichen Durchmessern bzw. Kenndaten auswählen. Die Verbindung der beiden Teile ist vorzugsweise lösbar und im verbundenen Zustand insbesondere ortsfest, d.h. eine relative Bewegung zwischen der Markereinheit und dem Fortsatz-Körper wird unterbunden.

Weitere Vorteile und Merkmale der Erfindung werden bei der folgenden detaillierten Beschreibung offenbart. Merkmale unterschiedlicher Ausführungsformen können miteinander kombiniert werden.
Figur 1 zeigt einen Fortsatzkörper mit daran ankoppelbaren Markern;
Figur 2a zeigt eine Vorderansicht des Fortsatzkörpers;
Figur 2b zeigt eine Seitenansicht des Fortsatzkörpers;
Figur 2c zeigt eine Rückansicht des Fortsatzkörpers;
Figur 3 zeigt ein erfindungsgemäßes Markersystem.
Figur 4 zeigt eine alternative Anordnung der Fortsätze auf einem Fortsatzkörper.

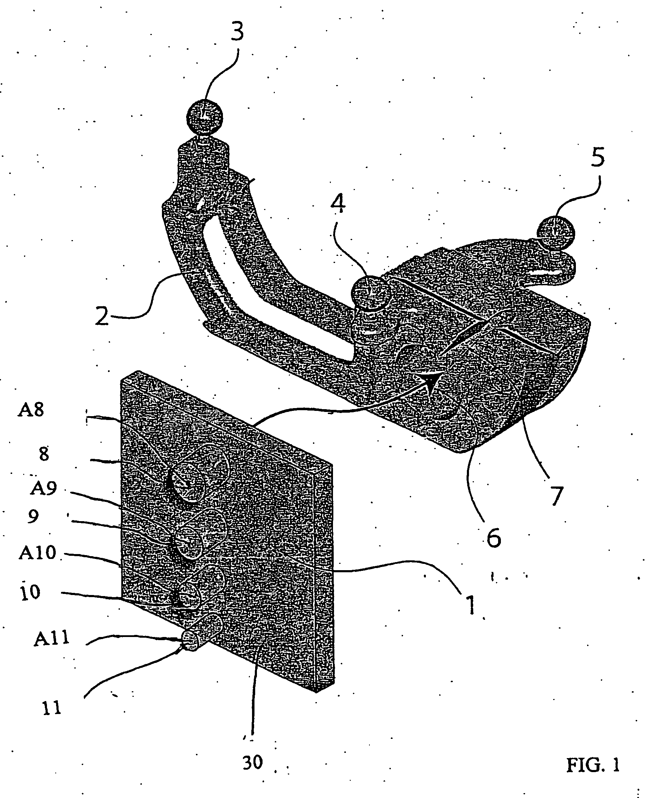

Der Fortsatzkörper 1 in Figur 1 weist mehrere Fortsätze auf. Der Fortsatz 8 hat die Achse A8. Der Fortsatz 9 hat die Achse A9, der Fortsatz 10 hat die Achse A10 und der Fortsatz 11 hat die Achse A11. Wie in der Figur 1 gezeigt, stehen die Fortsätze vorzugsweise von einer planen Ebene vor. Die Fortsätze sind bei der gezeigten Ausführungsform zylindrisch ausgebildet, haben also vorzugsweise einen konstanten Durchmesser entlang den Achsen. Der Durchmesser der Fortsätze unterscheidet sich vorzugsweise. Vorzugsweise sind die Fortsätze in einer Reihe angeordnet und der Abstand zwischen den Fortsätzen ist vorzugsweise mindestens so groß wie der Durchmesser der benachbarten Fortsätze, besonders bevorzugt größer als der Durchmesser des Fortsatzes mit dem größten Durchmesser. Vorzugsweise stehen die Fortsätze um einen gleichen Abstand von der Ebene 30, von der die Fortsätze vorstehen, vor.

Die Markereinheit 2 umfasst drei Marker mit vorzugsweise kugelförmiger Gestalt und vorzugsweise gleichem Durchmesser der Markerkugeln. Die relative Lage der Marker 3, 4 und 5 zueinander ist vorzugsweise bekannt und insbesondere charakteristisch für die Markereinheit. Somit kann aus der charakteristischen Anordnung der Marker 3, 4 und 5 erkannt werden, dass es sich um die Markereinheit 2 handelt.

Bei der in Figur 1 gezeigten Ausführungsform ist die Markereinheit 2 als Kalibriergerät ausgebildet, wobei Instrumente zu Kalibrierungszwecken an Ebenen der Markereinheit 2 angelegt werden können oder in Öffnungen eingeführt werden können, um eine Kalibrierung durchzuführen. Diese doppelte Funktion ohne Markereinheit 2 wird hierin jedoch nicht näher ausgeführt.

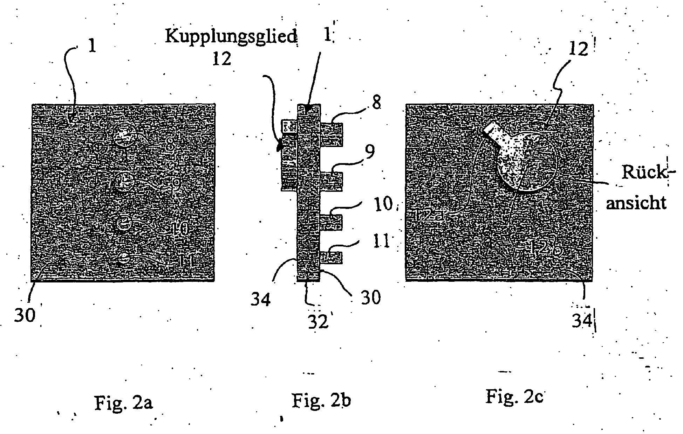

Figur 2a zeigt eine Vorderansicht des Fortsatzkörpers 1, wobei die Fortsätze 8, 9, 10 und 11 in Draufsicht als Kreise unterschiedlichen Durchmessers zu sehen sind.

Figur 2b zeigt eine Seitenansicht des Fortsatzkörpers 1, wobei rechts der Platte 32 mit der Vorderebene 30 die Fortsätze 8, 9, 10 und 11 von oben nach unten aufgereiht sind. Auf der Rückseite 34 der Platte 32 befindet sich ein Kupplungsglied 12, das von der planen Rückseite 34 vorsteht.

Das Kupplungsglied 12 ist näher in Figur 2c gezeigt. Die Figur 2c ist eine Rückansicht des Fortsatzkörpers 1. In Draufsicht ist dort das Kupplungsglied 12 gezeigt. Es besteht aus einem kreisförmigen Abschnitt 12b und aus einem von dem Kreisumfang des kreisförmigen Gliedes 12b vorstehenden, die Drehung blockierenden Fortsatz 12a.

Wie in Figur 1 gezeigt ist, wird der kreisförmige Abschnitt 12b in die kreisförmige Öffnung 6 der Markereinheit 2 eingeführt, wie durch den Pfeil B angezeigt ist. Außerdem wird der Fortsatz 12a in die komplementär ausgebildete Vertiefung 7 der Markereinheit 2 eingeführt. Der Fortsatz 12a verhindert somit in Zusammenwirkung mit der Vertiefung 7, dass der Fortsatzkörper 1 relativ zu der Markereinheit 2 sich drehen lässt. Vorzugsweise ist das Kupplungsglied 12 passgenau gestaltet, um eine stabile relative Lage zwischen dem Fortsatzkörper und der Markereinheit 2 zu gewährleisten. Zusätzlich oder alternativ kann auch eine rasterartige Kupplung vorgesehen werden.

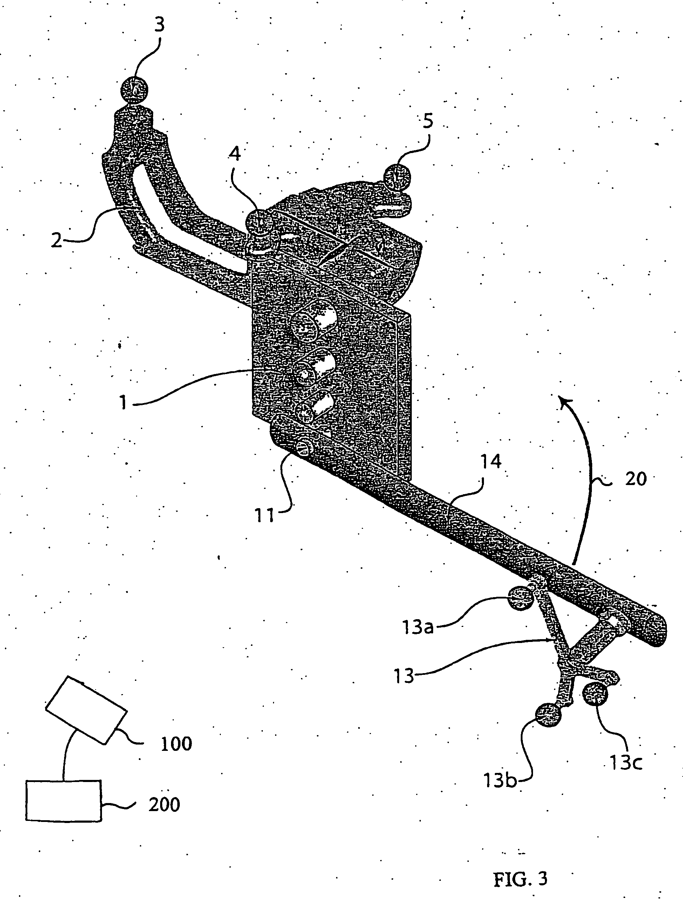

Figur 3 zeigt den Aufbau eines erfindungsgemäßen Markersystems. Gleiche Bezugszeichen bezeichnen wiederum gleiche Teile, wie bei den vorhergehenden Figuren.

In Figur 3 ist der Fortsatzkörper 1 mit der Markereinheit 2 lagefest gekoppelt, so dass sich der Fortsatzkörper 1 nicht relativ zu der Markereinheit 2 bewegen lässt. Weiter ist ein Beispiel für ein Instrument 14 gezeigt, das in Verbindung mit dem erfindungsgemäßen Markersystem vermessen werden kann. Die stabförmige Gestalt des Instruments 14 ist lediglich beispielhaft. Andere Ausgestaltungen des Instruments, wie beispielsweise eine dreieckige Gestalt sind natürlich möglich. Der Fortsatz 11 durchdringt eine zylindrische Öffnung (Durchgangsbohrung) des Instruments 14 in passgenauer Art und Weise, so dass die Achse des Fortsatzes 11 mit der Achse der zylindrischen Öffnung des Instruments 14 übereinstimmt. Eine Markereinrichtung 13 ist an einem Ende des Instruments 14 vorgesehen, das dem Ende gegenüberliegt, in dessen Nähe sich die zylindrische Öffnung befindet. Vorzugsweise sind die Instrumente so gestaltet, dass sich die Markereinrichtung 13 möglichst weit weg von der zu vermessenden zylindrischen Öffnung befindet, um den Mittelpunkt der Kreisbahn 20, auf der sich die Markereinrichtung 13 bewegt, genauer bestimmen zu können. Vorzugsweise beträgt der Abstand zwischen der Markereinrichtung 13 und dem Mittelpunkt der Drehbewegung ein Vielfaches des Durchmessers der zylindrischen Öffnung, insbesondere mehr als ein Fünffaches oder Zehnfaches. Die vorliegende Erfindung ist auch auf derartig geeignet gestaltete Instrumente mit Markereinrichtung gerichtet.

Bei den Markern 13a, 13b und 13c kann es sich um aktive Marker handeln, die Signale (z.B. Licht oder Ultraschall) aussenden. Vorzugsweise handelt es sich um passive Marker, die Signale reflektieren, die in ihre Richtung ausgestrahlt werden. Beispielsweise ist eine Infrarotlichtquelle vorgesehen, die kontinuierlich und vorzugsweise intermittierend Infrarotlicht abgibt, das von den Markerkugeln 13a, 13b, 13c, 3, 4 und 5 reflektiert wird. Die Kamera 100, die vorzugsweise als eine Kamera mit 2 räumlich getrennten Detektionselementen ausgebildet ist, empfängt das von den Markerkugeln 3, 4, 5, 13a, 13b und 13c ausgehende Signal (z.B. Infrarotlicht). Die Markerkugeln 3, 4 und 5 sowie die Markerkugeln 13a, 13b und 13c sind jeweils in einer charakteristischen Lagebeziehung zueinander. Diese charakteristische Lagebeziehung ist vorzugsweise bekannt. Beispielsweise spannen die Mittelpunkte der Markerkugeln 3, 4 und 5 ein Dreieck auf, dessen Seitenlängen und Winkel vorzugsweise bekannt sind. Entsprechend spannen die Mittelpunkte der Markerkugeln 13a, 13b und 13c ein Dreieck auf, dessen Seitenlängen und Winkel bekannt sind. Ist somit sowohl die Markeranordnung mit den Kugeln 3, 4 und 5 als auch die Markeranordnung mit den Kugeln 13a, 13b und 13c durch die Seitenlängen und Winkel des jeweiligen Dreiecks charakterisiert, so kann die Datenverarbeitungseinrichtung 200 aus den von der Kamera 100 übermittelten Datensignalen ermitteln, von welcher Markeranordnung die Signale stammen.

Um die Position der Achse der zylindrischen Öffnung sowie den Durchmesser der zylindrischen Öffnung des Instruments 14 zu bestimmen, wird wie folgt vorgegangen. Das Instrument 14 wird um die Achse des Fortsatzes 11 entlang der Kreisbahn 20 gedreht, so dass wenigstens drei Positionen von der Kamera 100 und somit auch von der Datenverarbeitungseinrichtung 200 erfasst werden. Die Datenverarbeitungseinrichtung 200 berechnet dann aus den Datensignalen von der Markereinrichtung 13 den Mittelpunkt der Kreisbahn, entlang der sich die Markereinrichtung 13 bewegt hat. Der Mittelpunkt dieser Kreisbahn stimmt mit der Achse des Fortsatzes 11 überein. Weiter ist die relative Lage zwischen der Markeranordnung mit den Kugeln 3, 4 und 5 und den einzelnen Fortsätzen, insbesondere dem Fortsatz 11 bekannt. Aus der Detektion der Signale von der Markeranordnung mit den Kugeln 3, 4 und 5 und aus der gespeicherten relativen Lage zwischen den Kugeln 3, 4 und 5 und den jeweiligen Fortsätzen, insbesondere dem Fortsatz 11 ist somit auch die Lage des Fortsatzes 11 bekannt. Die Datenverarbeitungseinrichtung kann somit überprüfen, ob der Mittelpunkt der Kreisbahn 20 mit der Lage der Achse des Fortsatzes 11 übereinstimmt. Ist dies der Fall, so ist somit die Lage der Achse der zylindrischen Öffnung bestimmt, denn diese stimmt mit dem Mittelpunkt der Kreisbahn überein. Die Lage kann beispielsweise in einem Bezugssystem beschrieben werden, in dem die Detektionseinrichtung ruht. Weiter ist durch die bekannte Lage des Mittelpunkts der Kreisbahn 20 und die bekannte Lage des Fortsatzes 11 bekannt, dass sich das Instrument um den Fortsatz 11 gedreht hat. Da die besonderen geometrischen Eigenschaften des Fortsatzes 11 in der Datenverarbeitungseinrichtung gespeichert sind, insbesondere der Durchmesser des Fortsatzes 11, ist somit auch der Durchmesser der zylindrischen Öffnung im Instrument 14 bekannt. Somit ist sowohl die Lage der zylindrischen Öffnung als auch deren Durchmesser mittels des erfindungsgemäßen Verfahrens und des erfindungsgemäßen Markersystems bestimmbar.

Figur 4 zeigt noch eine alternative Ausführungsform für einen Fortsatzkörper 1'. Der Fortsatzkörper 1' ist vorzugsweise ebenfalls mit einer (nicht gezeigten) Markereinrichtung verbunden, die beispielsweise wie die Anordnung 13 (Referenzstern) ausgebildet sein kann.

Bei der in Figur 4 gezeigten Ausführungsform geht von der Vorderfläche 30' des Fortsatzkörpers 1' ein zylindrischer Fortsatz hervor, dessen Durchmesser sich stufenweise mit zunehmendem Abstand von der Oberfläche 30' verkleinert. Vorzugsweise ist die Stufenhöhe größer als der Durchmesser des größten Fortsatzes 8'. Eine zylindrische Öffnung eines Instruments lässt sich auf den in Figur 4 gezeigten Fortsatzkörper 1' ebenfalls aufsetzen, wobei je nach Größe des Durchmessers der Öffnung das Instrument an einer der Stufen bzw. der Vorderebene 30' zur Anlage gebracht werden kann. Auch hier wird dann vorzugsweise eine Drehung des Instruments um den jeweils passenden Fortsatz 8', 9' oder 10' durchgeführt. Durch Detektion der Signale der Markeranordnung, die an dem Instrument angebracht ist, kann wiederum der Mittelpunkt der Drehbewegung bestimmt werden. Aus der bestimmten Lage des Mittelpunkts der Drehbewegung kann dann wiederum bestimmt werden, um welchen der Fortsätze 8', 9' oder 10' das Instrument gedreht wurde.

## Patentansprüche

1. Markersystem zum Bestimmen der Ausformung und Achslage eines rotationssymmetrischen Loches in einem Instrument (14), insbesondere einem medizinischen Instrument, an dem Instrumenten-Marker (13a, 13b, 13c) angebracht sind;
mit einem Körper (1, 2), an dem Körper-Marker (3, 4, 5) und rotationssymmetrische Fortsätze (8, 9, 10, 11) unterschiedlicher Ausformung vorgesehen sind;
mit einer Detektionseinrichtung (100)
zur Detektion von Signalen, die von den Körper-Markern (3, 4, 5) und von den Instrumenten-Markern (13a, 13b, 13c) des Instruments (14) ausgehen, sowie
zum Ausgeben von Datensignalen, die die detektierten Signale darstellen;
mit einer Datenverarbeitungseinrichtung (200)
zum Empfangen der Datensignale, die die relative Lage der Körper-Marker (3, 4, 5) relativ zur Detektionseinrichtung (100) und mindestens drei verschiedene relative Lagen der Instrumenten-Marker (13a, 13b, 13c) relativ zur Detektionseinrichtung (100) darstellen,
zum Speichern der relativen Lagen zwischen den Körper-Markern (3, 4, 5) und den Achsen (A8, A9, A10, A11) der rotationssymmetrischen Fortsätze (8, 9, 10, 11),
zum Speichern einer Zuordnung, die jeder Achse (A8, A9, A10, A11) mindestens ein Kenndatum zuordnet, das die Ausformung des rotationssymmetrischen Fortsatzes (8, 9, 10, 11) mit dieser Achse charakterisiert,
zum Berechnen, unter der Annahme, dass die mindestens drei verschiedenen relativen Lagen der Instrumenten-Marker (13a, 13b, 13c) auf einer Kreisbahn (20) liegen, der relativen Lage des Mittelpunktes der Kreisbahn (20) relativ zu der Detektionseinrichtung (100) basierend auf den Datensignalen, die die mindestens drei verschiedenen relativen Lagen (13a, 13b, 13c) darstellen,
zum Bestimmen, auf welcher der Achsen (A8, A9, A10, A11) die berechnete relative Lage des Mittelpunktes liegt, und zwar basierend auf den gespeicherten relativen Lagen der Achsen (A8, A9, A10, A11) relativ zu den Körper-Markern (3, 4, 5), den Datensignalen, die die relative Lage der Körper-Marker (3, 4, 5) relativ zu der Detektionseinrichtung (100) darstellen, und der berechneten relativen Lage des Mittelpunktes der Kreisbahn (20) relativ zu der Detektionseinrichtung (100), und
zum Bestimmen des mindestens einen Kenndatums, das der bestimmten Achse (A8, A9, A10, A11) zugeordnet ist, und zwar basierend auf der gespeicherten Zuordnung.

2. Markersystem nach Anspruch 1, bei welchem der Abstand zwischen den rotationssymmetrischen Fortsätzen (8, 9, 10, 11) größer ist als ein maximaler Durchmesser der Fortsätze (8, 9, 10, 11).

3. Markersystem nach Anspruch 1 oder 2, bei welchem die Fortsätze (8, 9, 10, 11) zylindrisch ausgebildet sind und das mindestens eine Kenndatum der Durchmesser oder Radius des Zylinders ist.

4. Markersystem nach einem der vorhergehenden Ansprüche, **dadurch gekennzeichnet, dass** der Körper (1, 2) so ausgebildet ist, dass die rotationssymmetrischen Fortsätze (8, 9, 10, 11) von einer planen oder konvexen Oberfläche vorstehen.

5. Markersystem nach einem der vorhergehenden Ansprüche, bei welchem die rotationssymmetrischen Fortsätze so angeordnet sind, dass die Achsen (A8, A9, A10, A11) der rotationssymmetrischen Fortsätze eine Trajektorie schneiden.

6. Markersystem nach Anspruch 5, bei welchem die Trajektorie eine gerade, gekrümmte Linie oder geschlossene Linie ist.

7. Markersystem nach einem der vorhergehenden Ansprüche, bei welchem der Körper (1, 2) einen Fortsatz-Körper (1), der mit den rotationssymmetrischen Fortsätzen (8, 9, 10, 11) versehen ist, und eine Markereinheit (2), die mit den Körper-Markern versehen ist, umfasst, wobei der Fortsatz-Körper (1) und die Markereinheit (2) lösbar miteinander verbindbar sind und im verbundenen Zustand eine ortsfeste relative Lage zueinander einnehmen.

8. Verfahren zum Bestimmen von Durchmesser und Achslage eines rotationssymmetrischen Loches in einem Instrument (14), insbesondere einem medizinischen Instrument, an dem Instrumenten-Marker (13a, 13b, 13c) angebracht sind und mit einem Körper (1, 2), an dem Körper-Marker (3, 4, 5) und rotationssymmetrische Fortsätze (8, 9, 10, 11) unterschiedlicher Ausformung vorgesehen sind, wobei das Verfahren die folgenden Schritte umfasst:
einer der Fortsätze (11), der in das Loch des Instruments (14) passt, wird in das Loch des Instruments eingeführt;
das Instrument wird um den, insbesondere passgenau eingeführten rotationssymmetrischen Fortsatz (11) gedreht;
Signale von den Körper-Markern (3, 4, 5) und von den Instrumenten-Markern (13a, 13b, 13c) werden von einer Detektionseinrichtung erfasst, wobei die erfassten Signale solche umfassen, die bei drei verschiedenen Lagen des Instruments während der Drehung um den eingeführten Fortsatz gewonnen wurden;
die detektierten Signale werden als Datensignale an eine Datenverarbeitungseinrichtung ausgegeben;
in der Datenverarbeitungseinrichtung werden als gespeicherte Daten die relative Lage zwischen den Körper-Markern (3, 4, 5) und den Achsen (A8, A9, A10, A11) der rotationssymmetrischen Fortsätze (8, 9, 10, 11) bereitgestellt, weiter wird eine Zuordnung bereitgestellt, die jeder Achse (A8, A9, A10, A11) mindestens ein Kenndatum zuordnet, das die Ausformung des jeweiligen rotationssymmetrischen Fortsatzes (8, 9, 10, 11) charakterisiert, der die jeweilige Achse hat;
die Datensignale, die die relative Lage der Körper-Marker (3, 4, 5) relativ zur Detektionseinrichtung (100) darstellen und von der Detektionseinrichtung ausgegeben werden, werden von der Datenverarbeitungseinrichtung empfangen;
Datensignale, die von der Detektionseinrichtung ausgegeben werden und die die mindestens drei verschiedenen relativen Lagen der Instrumenten-Marker (13a, 13b, 13c) relativ zur Detektionseinrichtung (100) darstellen, werden von der Datenverarbeitungseinrichtung empfangen;
basierend auf der Annahme, dass die mindestens drei verschiedenen relativen Lagen auf einer Kreisbahn liegen, wird die relative Lage des Mittelpunkts der Kreisbahn (20) relativ zu der Detektionseinrichtung (100) berechnet, und zwar basierend auf den Datensignalen, die die mindestens drei verschiedenen relativen Lagen (13a, 13b, 13c) darstellen;
die Achse (A8, A9, A10, A11) wird bestimmt, auf der die berechnete relative Lage des Mittelpunkts liegt, und zwar basierend auf den gespeicherten relativen Lagen der Achsen (A8, A9, A10, A11) relativ zu den Körper-Markern, den Datensignalen, die die relative Lage der Körper-Marker relativ zu der Detektionseinrichtung darstellen, und der berechneten relativen Lage des Mittelpunktes der Kreisbahn (20) relativ zu der Detektionseinrichtung (100); und
das mindestens eine Kenndatum, das der bestimmten Achse (A8, A9, A10, A11) zugeordnet ist, wird basierend auf der gespeicherten Zuordnung bestimmt.

9. Verwendung des Markersystems nach einem der Ansprüche 1 bis 7 zum Bestimmen von der Ausformung und Achslage eines rotationssymmetrischen Loches in einem Instrument gemäß dem Verfahren nach Anspruch 8.

## Claims

1. A marker system for determining the shaping and axial location of a rotationally symmetrical hole in an instrument (14), in particular a medical instrument, to which instrument markers (13a, 13b, 13c) are attached, comprising:
a body (1, 2) on which body markers (3, 4, 5) and rotationally symmetrical extensions (8, 9, 10, 11) of different shapings are provided;
a detection means (100)
for detecting signals emitted by the body markers (3, 4, 5) and by the instrument markers (13a, 13b, 13c) of the instrument (14) and
for outputting data signals which represent the detected signals;
a data processing means (200)
for receiving the data signals which represent the relative location of the body markers (3, 4, 5) relative to the detection means (100) and at least three different relative locations of the instrument markers (13a, 13b, 13c) relative to the detection means (100),
for storing the relative locations between the body markers (3, 4, 5) and the axes (A8, A9, A10, A11) of the rotationally symmetrical extensions (8, 9, 10, 11),
for storing an association which associates each axis (A8, A9, A10, A11) with at least one characteristic which characterises the shaping of the rotationally symmetrical extension (8, 9, 10, 11) having said axis,
for calculating - given the assumption that the at least three different relative locations of the instrument markers (13a, 13b, 13c) lie on a circular trajectory (20) - the relative location of the centre point of the circular trajectory (20) relative to the detection means (100), based on the data signals which represent the at least three different relative locations (13a, 13b, 13c),
for determining which of the axes (A8, A9, A10, A11) the calculated relative location of the centre point lies on, based on the stored relative locations of the axes (A8, A9, A10, A11) relative to the body markers (3, 4, 5), the data signals which represent the relative location of the body markers (3, 4, 5) relative to the detection means (100), and the calculated relative location of the centre point of the circular trajectory (20) relative to the detection means (100), and
for determining the at least one characteristic which is associated with the determined axis (A8, A9, A10, A11), based on the stored association.

2. The marker system according to claim 1, wherein the distance between the rotationally symmetrical extensions (8, 9, 10, 11) is larger than a maximum diameter of the extensions (8, 9, 10, 11).

3. The marker system according to claim 1 or 2, wherein the extensions (8, 9, 10, 11) are designed cylindrically, and the at least one characteristic is the diameter or radius of the cylinder.

4. The marker system according to any one of the preceding claims, **characterised in that** the body (1, 2) is designed such that the rotationally symmetrical extensions (8, 9, 10, 11) protrude from a planar or convex surface.

5. The marker system according to any one of the preceding claims, wherein the rotationally symmetrical extensions are arranged such that the axes (A8, A9, A 10, A11) of the rotationally symmetrical extensions intersect a trajectory.

6. The marker system according to claim 5, wherein the trajectory is a straight, curved or closed line.

7. The marker system according to any one of the preceding claims, wherein the body (1, 2) comprises an extension body (1) which is provided with the rotationally symmetrical extensions (8, 9, 10, 11), and a marker unit (2) which is provided with the body markers, wherein the extension body (1) and the marker unit (2) can be releasably connected to each other and, when connected, assume a spatially fixed location relative to each other.

8. A method for determining the diameter and axial location of a rotationally symmetrical hole in an instrument (14), in particular a medical instrument, to which instrument markers (13a, 13b, 13c) are attached and which comprises a body (1, 2) on which body markers (3, 4, 5) and rotationally symmetrical extensions (8, 9, 10, 11) of different shapings are provided, wherein the method comprises the following steps:
one of the extensions (11), which fits into the hole in the instrument (14), is inserted into the hole in the instrument;
the instrument is rotated around the rotationally symmetrical extension (11) which is in particular inserted in an exact fit;
signals from the body markers (3, 4, 5) and from the instrument markers (13a, 13b, 13c) are detected by a detection means, wherein the detected signals include signals obtained at three different locations of the instrument while it was being rotated around the inserted extension;
the detected signals are outputted as data signals to a data processing means;
in the data processing means, the relative location between the body markers (3, 4, 5) and the axes (A8, A9, A10, A11) of the rotationally symmetrical extensions (8, 9, 10, 11) are provided as stored data, and an association is provided which associates each axis (A8, A9, A10, A11) with at least one characteristic which characterises the shaping of the respective rotationally symmetrical extension (8, 9, 10, 11) having the respective axis;
the data signals which represent the relative location of the body markers (3, 4, 5) relative to the detection means (100) and are outputted by the detection means are received by the data processing means;
data signals which are outputted by the detection means and represent the at least three different relative locations of the instrument markers (13a, 13b, 13c) relative to the detection means (100) are received by the data processing means;
based on the assumption that the at least three different relative locations lie on a circular trajectory, the relative location of the centre point of the circular trajectory (20) relative to the detection means (100) is calculated, based on the data signals which represent the at least three different relative locations (13a, 13b, 13c);
the axis (A8, A9, A10, A11) on which the calculated relative location of the centre point lies is determined, based on the stored relative locations of the axes (A8, A9, A10, A11) relative to the body markers, the data signals which represent the relative location of the body markers relative to the detection means, and the calculated relative location of the centre point of the circular trajectory (20) relative to the detection means (100); and
the at least one characteristic associated with the determined axis (A8, A9, A10, A11) is determined, based on the stored association.

9. The use of the marker system according to any one of claims 1 to 7 for determining the shaping and axial location of a rotationally symmetrical hole in an instrument, in accordance with the method according to claim 8.

## Revendications

1. Système de marquage pour déterminer la forme et la position axiale d'un trou à symétrie de révolution dans un instrument (14), en particulier d'un instrument médical, sur lequel sont fixés des marqueurs d'instrument (13a, 13b, 13c), comportant :
un corps (1, 2) sur lequel sont prévus des marqueurs corporels (3, 4, 5) et des extensions à symétrie de révolution (8, 9, 10, 11) de forme différente,
un dispositif de détection (100) pour :
détecter des signaux émis par les marqueurs corporels (13a, 13b, 13c) et par les marqueurs d'instrument (13a, 13b, 13c) de l'instrument (14), ainsi que pour :
générer des signaux de données qui représentent les signaux détectés,
un dispositif de traitement de données (200) pour
recevoir les signaux de données qui représentent la position relative des marqueurs corporels (3, 4, 5) par rapport au dispositif de détection (100) et au moins trois positions relatives différentes des marqueurs d'instrument (3, 4, 5) par rapport au dispositif de détection (100),
mémoriser les positions relatives entre les marqueurs corporels (3, 4, 5) et les axes (A8, A9, A10, A11) des extensions à symétrie de révolution (8, 9, 10, 11) et
mémoriser une association qui associe une caractéristique à chaque axe (A8, A9, A10, A11), ladite caractéristique caractérisant la forme de l'extension à symétrie de révolution (8, 9, 10, 11) avec cet axe,
calculer, sur la base de l'hypothèse que les au moins trois positions relatives différentes des marqueurs d'instrument (13a, 13b, 13c) se situent sur une trajectoire circulaire (20), la position relative du point central de la trajectoire circulaire (20) par rapport au dispositif de détection (100), sur la base des signaux de données qui représentent les au moins trois positions relatives différentes (13a, 13b, 13c),
déterminer sur lequel des axes (A8, A9, A10, A11) se situe la position relative calculée du point central, c'est-à-dire en se basant sur les positions relatives mémorisées des axes (A8, A9, A10, A11) par rapport aux marqueurs corporels (3, 4, 5), sur les signaux de données qui représentent la position relative des marqueurs corporels (3, 4, 5) par rapport au dispositif de détection (100), et sur la position relative calculée du point central de la trajectoire circulaire (20) par rapport au dispositif de détection (100), et
déterminer la au moins une caractéristique qui est associée à l'axe (A8, A9, A10, A11) déterminé, c'est-à-dire en se basant sur l'association mémorisée.

2. Système de marquage selon la revendication 1, dans lequel la distance entre les extensions à symétrie de révolution (8, 9, 10, 11) est plus grande qu'un diamètre maximal des extensions (8, 9, 10, 11).

3. Système de marquage selon la revendication 1 ou 2, dans lequel les extensions (8, 9, 10, 11) sont formées de manière cylindrique et la au moins une caractéristique est le diamètre ou le rayon du cylindre.

4. Système de marquage selon l'une quelconque des revendications précédentes, **caractérisé en ce que** le corps (1, 2) est configuré de telle sorte que les extensions à symétrie de révolution (8, 9, 10, 11) font saillie à partir d'une surface plane ou convexe.

5. Système de marquage selon l'une quelconque des revendications précédentes, dans lequel les extensions à symétrie de révolution sont agencées de telle sorte que les axes (A8, A9, A10, A11) des extensions à symétrie de révolution coupent une trajectoire.

6. Système de marquage selon la revendication 5, dans lequel la trajectoire est une ligne droite, courbe ou fermée.

7. Système de marquage selon l'une quelconque des revendications précédentes, dans lequel le corps (1, 2) comporte un corps d'extension (1) muni des extensions à symétrie de révolution (8, 9, 10, 11), et une unité de marquage (2) munie des marqueurs corporels, dans lequel le corps d'extension (1) et l'unité de marquage (2) peuvent être couplés l'un à l'autre de manière amovible et prennent, à l'état couplé, une position relative fixe l'un par rapport à l'autre.

8. Procédé pour déterminer le diamètre et la position axiale d'un trou à symétrie de révolution dans un instrument (14), en particulier un instrument médical, sur lequel sont fixés des marqueurs d'instrument (13a, 13b, 13c) et comportant un corps (1, 2) sur lequel sont prévus des marqueurs corporels (3, 4, 5) et des extensions à symétrie de révolution (8, 9, 10, 11) de forme différente, le procédé comportant les étapes suivantes consistant à :
insérer dans le trou de l'instrument l'une des extensions (11) qui rentre dans le trou de l'instrument (14),
faire tourner l'instrument autour de l'extension à symétrie de révolution (11) insérée de manière précise,
détecter, au niveau d'un dispositif de détection, des signaux provenant des marqueurs corporels (3, 4, 5) et des marqueurs d'instrument (13a, 13b, 13c), les signaux détectés incluant les signaux acquis pour les trois positions différentes de l'instrument lors de la rotation autour de l'extension insérée,
délivrer les signaux détectés sous forme de signaux de données en sortie d'un dispositif de traitement de données,
déterminer, dans le dispositif de traitement de données, la position relative entre les marqueurs corporels (3, 4, 5) et les axes (A8, A9, A10, A11) des extensions à symétrie de révolution (8, 9, 10, 11) sous forme de données mémorisées, puis préparer une association qui associe au moins une caractéristique à chaque axe (A8, A9, A10, A11), ladite caractéristique caractérisant la forme de l'extension à symétrie de révolution (8, 9, 10, 11) respective que possède l'axe respectif,
recevoir, au niveau du dispositif de traitement de données, les signaux de données qui représentent la position relative des marqueurs corporels (3, 4, 5) par rapport au dispositif de détection (100) et générés par le dispositif de détection,
recevoir, au niveau du dispositif de traitement de données, des signaux de données générés par le dispositif de détection et représentant les au moins trois positions relatives différentes des marqueurs d'instrument (13a, 13b, 13c) par rapport au dispositif de détection (100),
sur la base de l'hypothèse que les au moins trois positions relatives différentes se situent sur une trajectoire circulaire, calculer la position relative du point central de la trajectoire circulaire (20) par rapport au dispositif de détection (100), c'est-à-dire en se basant sur les signaux de données représentant les au moins trois positions relatives différentes (13a, 13b, 13c),
déterminer l'axe (A8, A9, A10, A11) sur lequel se situe la position relative calculée du point central, c'est-à-dire en se basant sur les positions relatives mémorisées des axes (A8, A9, A10, A11) par rapport aux marqueurs corporels, sur les signaux de données représentant la position relative des marqueurs corporels par rapport au dispositif de détection, et sur la position relative calculée du point central de la trajectoire circulaire (20) par rapport au dispositif de détection (100), et
déterminer, sur la base de l'association mémorisée, la au moins une caractéristique qui est associée à l'axe (A8, A9, A10, A11) déterminé.

9. Utilisation du système de marquage selon l'une quelconque des revendications 1 à 7 pour déterminer la forme et la position axiale d'un trou à symétrie de révolution dans un instrument selon le procédé de la revendication 8.
